# EUROPÄISCHE PATENTANMELDUNG

(11) **EP 3 702 345 A1**
(43) Veröffentlichungstag der Anmeldung: **02.09.2020**
(21) Anmeldenummer: 19159439.9
(22) Anmeldetag: 26.02.2019
(51) Int. Cl.: C07C 5/327, C07C 7/144, C07C 11/08

(54) **VERFAHREN UND ANLAGE ZUR GEWINNUNG EINES BUTYLENPRODUKTS**

(71) Anmelder: Linde GmbH, 82049 Pullach (DE)
(72) Erfinder: Fritz, Helmut, 81375 München (DE)
(74) Vertreter: Frommberger, Moritz

(57) **Zusammenfassung**

Die vorliegende Erfindung betrifft ein Verfahren (100) zur Herstellung eines Butylenprodukts (9), bei dem unter Einsatz einer Butandehydrierung (10), der ein Butan und Wasserstoff enthaltender Reaktionseinsatz (1) unterworfen wird, ein Butan, Butylen und Wasserstoff enthaltendes Komponentengemisch (2) bereitgestellt wird, wobei das Komponentengemisch (2) oder ein Teil hiervon als ein erster Trenneinsatz einer ersten Membrantrennung (40) unterworfen wird, mittels derer ein gegenüber dem ersten Trenneinsatz an Wasserstoff angereichertes und erstes Permeat (3) sowie ein gegenüber dem ersten Trenneinsatz an Wasserstoff abgereichertes, Wasserstoff, Butan und Butylen enthaltendes erstes Retentat (4) gebildet werden, wobei das erste Retentat (4) oder ein Teil hiervon als zweiter Trenneinsatz einer zweiten Membrantrennung (50) unterworfen wird, in der ein zumindest den überwiegenden Teil des Wasserstoffs des zweiten Trenneinsatzes enthaltendes zweites Permeat (6) sowie ein zumindest den überwiegenden Teil des Butans und des Butylens des zweiten Trenneinsatzes enthaltendes zweites Retentat gebildet werden, wobei die erste Membrantrennung (40) unter Einsatz eines Butan enthaltenden Spülgases (5) durchgeführt wird und das erste Permeat (3) als mit Butan des Spülgases (5) beaufschlagtes Permeat (3) erhalten wird und/oder die zweite Membrantrennung (50) unter Einsatz des Butan enthaltenden Spülgases (5) durchgeführt wird und das zweite Permeat (6) als mit Butan des Spülgases (5) beaufschlagtes Permeat (6) erhalten wird, und wobei das mit Butan des Spülgases (5) beaufschlagte erste Permeat (3) und/oder das mit Butan des Spülgases beaufschlagte zweite Permeat (3) oder ein oder mehrere Teile hiervon bei der Bildung des Reaktionseinsatzes (1) verwendet wird. Eine entsprechende Anlage ist ebenfalls Gegenstand der vorliegenden Erfindung.

## Beschreibung

Die Erfindung betrifft ein Verfahren und eine Anlage zur Herstellung eines Butylenprodukts gemäß den Oberbegriffen der unabhängigen Patentansprüche

### Stand der Technik

Butylene (Butene; nachfolgend wird ungeachtet der möglicherweise vorhandenen Butylenisomere lediglich der Begriff "Butylen" im Singular verwendet) werden herkömmlicherweise überwiegend durch Dampfspalten (engl. Steam Cracking) von Kohlenwasserstoffeinsätzen und weitere Umwandlungsverfahren im Zuge von Raffinerieprozessen hergestellt. In diesen Fällen stellt Butylen ein Nebenprodukt dar, das allerdings nur in vergleichsweise geringer Menge anfällt. Ein alternatives Verfahren zur Herstellung von Butylenen ist die Butandehydrierung.

Die (katalytische) Butandehydrierung ist ein allgemein bekanntes Verfahren der petrochemischen Industrie, das gewisse Gemeinsamkeiten zur Propandehydrierung aufweist, wie sie beispielsweise im Artikel "Propene" in Ullmann's Encyclopedia of Industrial Chemistry, Onlineausgabe 16. September 2013, DOI: 10.1002/14356007.a22_211 .pub3, insbesondere Abschnitt 3.3.1, "Propane Dehydrogenation", beschrieben ist.

Bei der Butandehydrierung handelt es sich wie bei der Propandehydrierung um eine endotherme Gleichgewichtsreaktion, die im Allgemeinen an Edel- oder Schwermetallkatalysatoren, umfassend beispielsweise Platin oder Chrom, durchgeführt wird. Die Dehydrierungsreaktion ist ausgesprochen selektiv. Für kommerziell verfügbare Prozesse werden Gesamtausbeuten von ca. 90% genannt. Ungeachtet dieser hohen Selektivität entstehen neben dem abgespaltenen Wasserstoff jedoch typischerweise auch geringere Mengen an Kohlenwasserstoffen mit einem, zwei und drei, sowie mehr als vier Kohlenstoffatomen als Nebenprodukte. Diese Nebenprodukte sowie bei der Butandehydrierung nicht umgesetztes Butan müssen zur Gewinnung eines Butylenprodukts abgetrennt werden.

Dampfspaltverfahren und Raffinerieprozesse, in denen Butylen gebildet wird, sind ebenfalls umfangreich in der Literatur beschrieben, beispielsweise im Artikel "Ethylene" in Ullmann's Encyclopedia of Industrial Chemistry, Onlineveröffentlichung 15. April 2009, DOI: 10.1002/14356007.a10_045.pub3, und im Artikel "Oil Refining" in Ullmann's Encyclopedia of Industrial Chemistry, Onlineveröffentlichung 15. Januar 2007, DOI: 10.1002/14356007.a18_051 .pub2.

Auch in Dampfspaltverfahren und Raffinerieprozessen werden Butylen enthaltende Komponentengemische gebildet, die zur Gewinnung eines Butylenprodukts entsprechend bearbeitet werden müssen. Die vorliegende Erfindung eignet sich grundsätzlich für alle Verfahren und Anlagen, in denen Komponentengemische gebildet werden, die Butylen sowie insbesondere tiefer als Butylen siedende Komponenten enthalten.

In kommerziellen Anlagen zur Butandehydrierung werden bei der Bearbeitung eines entsprechenden Komponentengemischs nach einer entsprechenden Vorbereitung, beispielsweise einer Verdichtung und Kohlendioxidentfernung, typischerweise zunächst Kohlenwasserstoffe mit zwei Kohlenstoffatomen und leichter siedende Verbindungen von Kohlenwasserstoffen mit drei und mehr Kohlenstoffatomen kryogen abgetrennt (sogenannte Deethanisierung). Weitere Trennschritte umfassen die Bildung einer überwiegend oder ausschließlich Butan und Butylen enthaltenden Fraktion sowie die Auftrennung dieser Fraktion. Vergleichbare Verfahren kommen auch zur Bearbeitung von Komponentengemischen zum Einsatz, die in Dampfspaltverfahren und Raffinerieprozessen gebildet werden.

Die aus dem Stand der Technik bekannten kryogenen Verfahren zur Bearbeitung entsprechender Komponentengemische sind durch einen ausgesprochen hohen Investitionsaufwand gekennzeichnet, der insbesondere aus den Maschinen zur Kälteerzeugung und die Apparate für die Kälteübertragung resultiert. Aufgrund des festzustellenden (positiven) Skaleneffekts (engl. Economy of Scale) handelt es sich bei den kryogenen Verfahren für Anlagen großer Kapazität um die Trennverfahren der Wahl. Für Anlagen geringerer Kapazität erhöhen sich die spezifischen Investitionskosten für kryogene Verfahren jedoch unverhältnismäßig.

Es besteht daher der Bedarf nach Alternativen zur trenntechnischen Bearbeitung von Butylen und tiefer als Butylen siedende Komponenten enthaltenden Komponentengemischen, insbesondere wenn diese Komponentengemische in einer Butandehydrierung und in vergleichsweise geringen Mengen anfallen.

### Offenbarung der Erfindung

Vor diesem Hintergrund schlägt die vorliegende Erfindung ein Verfahren und eine Anlage zur Herstellung von Butylen mit den jeweiligen Merkmalen der unabhängigen Patentansprüche vor. Bevorzugte Ausgestaltungen sind Gegenstand der abhängigen Patentansprüche sowie der nachfolgenden Beschreibung.

Fluide beliebiger Art werden hier generell als "reich" an einer oder mehreren enthaltenen Komponenten bezeichnet, wenn der Gehalt an der einen oder den mehreren Komponenten wenigstens 50%, 60%, 70%, 80%, 90%, 95%, 99% oder 99,5% auf molarer, Gewichts- oder Volumenbasis beträgt. Sie werden hingegen als "arm" an der einen oder den mehreren Komponenten bezeichnet, wenn der Gehalt an der einen oder den mehreren Komponenten höchstens 30%, 20%, 10%, 5%, 1% oder 0,5% auf molarer, Gewichts- oder Volumenbasis beträgt. Sie können im hier verwendeten Sprachgebrauch "angereichert" oder "abgereichert" an einer oder mehreren Komponenten sein, wobei sich diese Begriffe auf einen entsprechenden Gehalt in einem Ausgangsfluid beziehen, aus dem das jeweilige Fluid gebildet wurde. Das Fluid wird als angereichert bezeichnet, wenn es zumindest den 2-fachen, 5-fachen, 10-fachen, 100-fachen oder 1.000-fachen Gehalt der einen oder der mehreren Komponenten, bezogen auf das Ausgangsfluid, aufweist. Das Fluid wird hingegen als abgereichert bezeichnet, wenn es höchstens den 0,5-fachen, 0,1-fachen, 0,01-fachen oder 0,001-fachen Gehalt der einen oder der mehreren Komponenten, bezogen auf das Ausgangsfluid, aufweist. Ein "überwiegend" eine oder mehrere Komponenten enthaltendes Fluid ist reich an dieser oder diesen im Sinne der obigen Definition.

Ein Fluid (die Bezeichnung Fluid wird stellvertretend auch für entsprechende Ströme, Fraktionen usw. verwendet) ist im hier verwendeten Sprachgebrauch von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Stoffstrom kann auch beispielsweise einfach dadurch gebildet werden, dass er aus einem Speicherbehälter abgezogen bzw. von einem anderen Stoffstrom abgetrennt wird.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Die vorliegende Erfindung beruht insbesondere auf der Erkenntnis, dass für bekannte Membrantrennverfahren (praktisch) kein Skaleneffekt festzustellen ist, weshalb sie im Allgemeinen für hohe Anlagenkapazitäten keine wirtschaftlichen Lösungen darstellen, dagegen für kleine Anlagen oft kostengünstige Lösungen sind. Membrantrennverfahren unterscheiden sich grundsätzlich von kryogenen Destillationsverfahren typischerweise dahingehend, dass sie nur für Grob-(Bulk-)Abtrennungen geeignet sind, aber keinen hochreinen Produkte liefern können.

Die vorliegende Erfindung schlägt nun vor diesem Hintergrund ein einfaches und kostengünstiges Verfahren zur Abtrennung einer butylenhaltigen Fraktion bzw. eines Butylenprodukts aus einem unter Einsatz einer Butandehydrierung gebildeten Komponentengemisch vor, wobei das Komponentengemisch insbesondere in vergleichsweise geringer Produktionsmenge gebildet wird bzw. die Butandehydrierung eine vergleichsweise geringe Produktionskapazität besitzt. Unter einer "geringen Produktionsmenge" bzw. einer "geringen Produktionskapazität" wird dabei hier eine Produktionsmenge von 20 bis 120 kta (Kilotonnen pro Jahr), insbesondere 35 bis 80 kta, beispielsweise ca. 50 kta, verstanden.

Die im Rahmen der vorliegenden Erfindung vorgeschlagene Trennung erweist sich insbesondere hinsichtlich der Investitionskosten für entsprechend geringe Produktionskapazitäten als deutlich vorteilhafter als herkömmliche kryogene Trennsequenzen, da auf aufwendige Apparate und Maschinen verzichtet werden kann. Zugleich kann, wie auch nachfolgend noch im Detail erläutert, im Rahmen der vorliegenden Erfindung in der Trennung ein Reaktionseinsatz bereitgestellt werden, der Butan und Wasserstoff in für die Butandehydrierung geeigneten Mengen aufweist. Auf externe Zudosierungen kann daher verzichtet werden.

Die vorliegende Erfindung eignet sich insbesondere für die Verwendung in kleineren Butandehydrierungsanlagen, die zur Produktionssteigerung in bestehenden Anlagen, beispielsweise Anlagen zum Dampfspalten, nachgerüstet werden können. Aufgrund der erfindungsgemäß vorgeschlagenen Wasserstoffabtrennung können die verbleibenden Gasgemische in derartigen vorhandenen Anlagen bzw. den dort bereitgestellten Trenneinrichtungen bearbeitet werden und überlasten diese nicht. Es sind daher keine weiteren Umrüstungsmaßnahmen erforderlich.

Die vorliegende Erfindung schlägt insgesamt ein Verfahren zur Herstellung eines Butylenprodukts vor, bei dem unter Einsatz einer Butandehydrierung, der ein Butan und Wasserstoff enthaltender Reaktionseinsatz unterworfen wird, ein Butan, Butylen und Wasserstoff enthaltendes Komponentengemisch bereitgestellt wird. Wie bereits zuvor angesprochen, kann das erfindungsgemäße Verfahren dabei insbesondere in ein Gesamtverfahren bzw. eine Anlage integriert werden, in der weitere stoffumwandelnde Verfahrensschritte und Trennschritte durchgeführt werden, beispielsweise ein oder mehrere Dampfspaltschritte und diesem zugeordnete Trennschritte. Das erfindungsgemäß hergestellte Butylenprodukt kann dabei insbesondere auch unter Verwendung entsprechender weiterer Trennschritte gewonnen werden.

Die vorliegende Erfindung sieht vor, dass das Komponentengemisch oder ein Teil hiervon als ein erster Trenneinsatz einer ersten Membrantrennung unterworfen wird, mittels derer ein gegenüber dem ersten Trenneinsatz an Wasserstoff angereichertes erstes Permeat sowie ein gegenüber dem ersten Trenneinsatz an Wasserstoff abgereichertes, Wasserstoff, Butan und Butylen enthaltendes erstes Retentat gebildet werden. Das erste Permeat enthält dabei vorteilhafterweise kein oder nur geringe Mengen an Butan und Butylen, die in dem der ersten Membrantrennung zugeführten ersten Trenneinsatz enthalten sind. Wie erwähnt, sind Membrantrennverfahren typischerweise nicht dafür eingerichtet, eine vollständige Abtrennung von Komponenten zu erzielen, so dass das erste Retentat noch beträchtliche Mengen des in dem ersten Trenneinsatz enthaltenen Wasserstoffs enthält. Für dessen weitere Abtrennung wird die nachfolgend noch erläuterte zweite Membrantrennung verwendet.

Die erste (und die nachfolgend noch erläuterte zweite) Membrantrennung kann bzw. können im Rahmen der vorliegenden Erfindung unter Verwendung von aus dem Stand der Technik grundsätzlich bekannten Membrantrenneinrichtungen durchgeführt werden. Zu weiteren Details sei auf Fachliteratur verwiesen.

Unter einem "Permeat" wird hier ein Gas oder Gasgemisch verstanden, das überwiegend oder ausschließlich Komponenten aufweist, die von einer in einer Membrantrennstufe eingesetzten Membran nicht oder überwiegend nicht zurückgehalten werden, die also die Membran (im Wesentlichen oder zumindest bevorzugt) ungehindert passieren. Entsprechend handelt es sich bei einem "Retentat" um ein Gas oder Gasgemisch, das überwiegend oder ausschließlich Komponenten aufweist, die von der in der Membrantrennstufe eingesetzten Membran vollständig oder zumindest überwiegend zurückgehalten werden.

Im Rahmen der vorliegenden Erfindung wird das erste Retentat oder ein Teil hiervon als zweiter Trenneinsatz einer zweiten Membrantrennung unterworfen, in der ein zumindest den überwiegenden Teil des Wasserstoffs des zweiten Trenneinsatzes enthaltendes zweites Permeat sowie ein zumindest den überwiegenden Teil des Butans und des Butylens des zweiten Trenneinsatzes enthaltendes zweites Retentat gebildet werden. In der zweiten Membrantrennung wird, mit anderen Worten, der Wasserstoff aus dem zweiten Trenneinsatz überwiegend oder vollständig entfernt, so dass ein vorteilhafterweise im Wesentlichen schwerere Komponenten, insbesondere Butan und Butylen, enthaltendes Retentat gebildet wird. Dieses zweite Retentat kann dann zur Gewinnung des Butylenprodukts weiter aufbereitet werden.

Im Rahmen der vorliegenden Erfindung können grundsätzlich zwei unterschiedliche, je nach den vorliegenden Gegebenheiten vorteilhafte Alternativen zum Einsatz kommen. So kann die erste Membrantrennung unter Einsatz eines Butan enthaltenden Spülgases durchgeführt werden und das erste Permeat damit als mit Butan des Spülgases beaufschlagtes Permeat erhalten werden. Ein entsprechendes Spülgas wird in der Membrantrennung auf der Permeatseite entlang der verwendeten Membran geführt und ermöglicht es, eine größere Menge bzw. einen größeren Anteil über die Membran zu gewinnen, da durch die Membran tretende leichtere Komponenten kontinuierlich abgeführt werden können und damit ein Konzentrations- bzw. Partialdruckgefälle über die Membran hinweg aufrecht erhalten werden kann. Als das Butan enthaltende Spülgas wird dabei insbesondere Butan oder ein an Butan reiches, jedenfalls aber vorteilhafterweise an Wasserstoff armes oder freies, Gas oder Gasgemisch verwendet, um die genannten vorteilhaften Effekte in der Membrantrennung zu erzielen. In jedem Fall stammt das Butan in dem mit Butan des Spülgases beaufschlagten Permeat aus dem Spülgas, und nicht oder nur zu geringen Anteilen aus dem ersten Trenneinsatz.

Alternativ oder zusätzlich ist es im Rahmen der vorliegenden Erfindung aber auch möglich, die zweite Membrantrennung unter Einsatz des Butan enthaltenden Spülgases durchzuführen wird und damit das zweite Permeat als mit Butan des Spülgases beaufschlagtes Permeat zu erhalten wird. Hierzu gilt im Wesentlichen das bereits zu dem Einsatz des Butan enthaltenden Spülgases in der ersten Membrantrennung Gesagte.

Im Rahmen der vorliegenden Erfindung ist schließlich vorgesehen, das mit Butan des Spülgases beaufschlagte erste Permeat und/oder das mit Butan des Spülgases beaufschlagte zweite Permeat oder ein oder mehrere Teile hiervon bei der Bildung des Reaktionseinsatzes zu verwenden. Wird eines der Permeate, d.h. das erste oder das zweite Permeat, hingegen nicht als mit Butan des Spülgases beaufschlagtes Permeat gewonnen, wird also in der entsprechenden ersten oder zweiten Membrantrennung kein entsprechendes Spülgas eingesetzt, wird dieses typischerweise nicht bei der Bildung des Reaktionseinsatzes verwendet sondern, wie auch nachfolgend noch erläutert, insbesondere in einem oder mehreren Reaktoren unterfeuert bzw. einem Brenngasnetzwerk in einer entsprechenden Anlage zugeführt.

Der besondere Vorteil, ein mit Butan eines Butan enthaltenden Spülgases beaufschlagtes Permeat bei der Bildung des Reaktionseinsatzes zu verwenden, besteht darin, dass das entsprechende mit Butan beaufschlagte Permeat bereits die in einem entsprechenden Reaktionseinsatz benötigten Komponenten, Butan und Wasserstoff, enthält, und damit keine separate Zudosierung erfolgen muss. Im Rahmen der vorliegenden Erfindung ergibt durch die Verwendung des Spülgases der doppelte Vorteil, dass die entsprechende Membrantrennung vorteilhaft beeinflussen und zugleich ein für einen Einsatz in einem Trenneinsatz direkt verwendbares Gasgemisch bereitstellen lässt. Insbesondere kann, weil der in einem entsprechenden Reaktionseinsatz in der erfindungsgemäßen Weise bereitgestellte Wasserstoff nicht aus externen Quellen sondern aus einer Membrantrennung stammt, auf zusätzliche, ggf. ansonsten erforderliche Reinigungsschritte verzichtet werden.

Im Rahmen der vorliegenden Erfindung enthält der der Butandehydrierung unterworfene Reaktionseinsatz insbesondere 45 bis 95 Volumenprozent Butan und 1 bis 50 Volumenprozent Wasserstoff. Ein entsprechender Wasserstoffanteil kann vorteilhafterweise teilweise oder vollständig durch den erfindungsgemäß in dem oder den Membrantrennnschritten in das entsprechend verwendete Permeat übergehenden Wasserstoff gedeckt werden.

Das in der Butandehydrierung gebildete Komponentengemisch und der erste Trenneinsatz enthalten vorteilhafterweise 40 bis 60 Volumenprozent Butan, 20 bis 30 Volumenprozent Butylen und 25 bis 30 Volumenprozent Wasserstoff. In der Butandehydrierung wird damit zusätzlicher (überschüssiger) Wasserstoff gebildet, der in dem Anteil, in dem er nicht in die Butandehydrierung zurückgeführt wird, in vorteilhafter Weise für andere Zwecke genutzt werden kann.

In der ersten Membrantrennung erfolgt, wie bereits erwähnt, eine teilweise Abreicherung des ersten Trenneinsatzes an Wasserstoff, so dass das das erste Retentat und der zweite Trenneinsatz im Rahmen der vorliegenden Erfindung insbesondere 10 bis 20 Volumenprozent Wasserstoff aufweisen.

Durch den Einsatz der zweiten Membrantrennung erfolgt eine weitere Wasserstoffabreicherung, so dass das zweite Retentat nur noch geringe Anteile von Wasserstoff von 5 bis 15 Volumenprozent Wasserstoff aufweist.

Vorteilhafterweise wird in dem erfindungsgemäßen Verfahren das Komponentengemisch oder dessen der ersten Membrantrennung unterworfener Teil einer Abkühlung und Verdichtung unterworfen, bevor es oder er der Membrantrennung unterworfen wird. Die Abkühlung erfolgt dabei insbesondere durch Wärmetausch mit dem Reaktionseinsatz oder einem Teil hiervon, bevor dieser der Butandehydrierung unterworfen wird. In diese Ausgestaltung erlaubt das erfindungsgemäß vorgeschlagene Verfahren eine besonders vorteilhafte Wärmerückgewinnung.

Die Verdichtung des Komponentengemischs oder dessen der ersten Membrantrennung unterworfenen Teils erfolgt im Rahmen der vorliegenden Erfindung vorteilhafterweise auf ein Druckniveau von 2 bis 38 bar, 6 bis 20 bar, 8 bis 18 bar, 10 bis 14 bar, 10 bis 16 bar oder 12 bis 14 bar. Dieses Druckniveau richtet sich insbesondere nach dem erforderlichen Abgabedruck des zweiten Retentats bzw. auf einem erforderlichen Eintrittsdruck des oder der Verfahrensschritte, dem oder denen das zweite Retentat unterworfen werden soll. Das Druckniveau richtet sich ferner nach dem Volumenanteil des Wasserstoffs in dem Komponententgemisch aus der Butandehydrierung, weil sich der vorteilhafte Abtrenndruck in der ersten und der zweiten Membrantrennung wiederum von einem entsprechenden Volumenanteil abhängig. Ferner wird das Druckniveau dann, wenn in der ersten und der zweiten Membrantrennung Wasserstoff oder eine wasserstoffreiche Fraktion als Permeat gewonnen wird, der Wärmegewinnung verfeuert werden soll, auch nach dem hierzu erforderlichen Druck, insbesondere dann, wenn eine Einspeisung in ein Heizgasnetzwerk erfolgen soll. Ein weiterer Einflussfaktor ist die Außentemperatur, weil Kohlenwasserstoffe mit drei und ggf. mehr Kohlenstoffatomen in der ersten und der zweiten Membrantrennung nicht kondensieren sollen. Je nach der Ausgestaltung der in dem ersten und dem zweiten Membrantrennschritt eingesetzten Membranen kann in Abhängigkeit vom Partialdruck entsprechender Komponenten auch eine Begleitheizung vorteilhaft sein. Schließlich ist ein weiterer druckbeeinflussender Faktor der verwendete Verdichter. Vorteilhafterweise sollte dieser nicht mehr als zwei Zwischenkühlstufen aufweisen.

Als Verdichter wird vorteilhafterweise ein mit einem Elektromotor angetriebener Verdichter, insbesondere ein dreistufiger Turboverdichter, eingesetzt, da durch den elektrischen Antrieb aufwendige Turbinen und eine ebenso aufwendige Dampfsystemanbindung eingespart werden können.

Die Einspeisung des zweiten Retentats in dem oder die weiteren Verfahrensschritte, insbesondere zur Dampfspaltung oder einem oder mehreren dieser nachgeschalteten Trennschritten, erfolgt vorteilhafterweise auf einem Druckniveau von 1 bis 38 bar, so dass bei geeigneter Wahl der Verdichtung des Rohgases aus der Butandehydrierung keine weiteren Verdichtungsschritte erforderlich sind.

Insbesondere kann im Rahmen der vorliegenden Erfindung nur die erste Membrantrennung unter Einsatz des Butan enthaltenden Spülgases durchgeführt werden und damit nur das erste Permeat als ein mit Butan des Spülgases beaufschlagtes Permeat erhalten werden. Alternativ dazu kann nur die zweite Membrantrennung unter Einsatz des Butan enthaltenden Spülgases durchgeführt werden und nur das zweite Permeat als mit Butan des Spülgases beaufschlagtes Permeat erhalten werden. In beiden Fällen kann das jeweils nicht mit Butan des Spülgases beaufschlagte Permeat oder ein Teil hiervon in einem oder mehreren für die Butandehydrierung verwendeten Reaktoren zur Beheizung verfeuert werden. Hierzu kann beispielsweise eine Einspeisung in ein Heizgasnetzwerk erfolgen, wie bereits erwähnt. Alternativ dazu ist auch eine andere Nutzung eines entsprechenden, insbesondere überwiegend oder ausschließlich Wasserstoff aufweisenden Permeats möglich. In jedem Fall kann auf diese Weise der in der Butandehydrierung gebildete Wasserstoff einer vorteilhaften Verwendung zugeführt werden.

Des jeweils nicht mit Butan des Spülgases beaufschlagte Permeat wird für eine entsprechende Verwendung in der jeweiligen ersten oder zweiten Membrantrennung vorteilhafterweise auf einem Druckniveau von 3 bis 7 bar bereitgestellt und kann auf diese Weise insbesondere in ein Heizgasnetzwerk, das auf einem entsprechenden Druckniveau betrieben wird, eingespeist werden.

Die vorliegende Erfindung stellt auch eine Anlage zur Verfügung, deren Merkmale in dem entsprechenden unabhängigen Patentanspruch angegeben sind. Zu Merkmalen und Vorteilen der erfindungsgemäß vorgeschlagenen Anlage sei auf die zuvor bereits bezüglich des erläuterten Verfahrens und seiner vorteilhaften Ausgestaltung in erläuterten Merkmale und Vorteile ausdrücklich verwiesen. Entsprechendes gilt insbesondere für eine Anlage gemäß einer besonders bevorzugten Ausführungsformen der vorliegenden Erfindung, welche Mittel aufweist, die zur Durchführung eines entsprechenden Verfahrens eingerichtet sind.

Die Erfindung wird nachfolgend unter Bezugnahme auf die beigefügte Zeichnungen näher erläutert, in der eine bevorzugte Ausführungsform der vorliegenden Erfindung veranschaulicht ist.

### Kurze Beschreibung der Zeichnungen

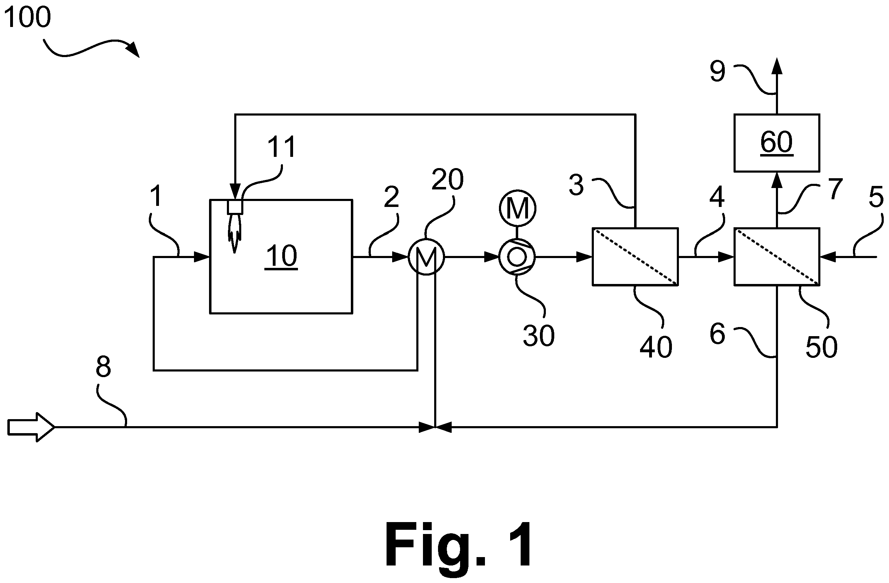

Figur 1 veranschaulicht einen gemäß einer Ausführungsform der Erfindung ausgestaltetes Verfahren in stark vereinfachter, schematischer Darstellung.

### Ausführliche Beschreibung der Zeichnungen

In Figur 1 ist ein erfindungsgemäß ausgestaltetes Verfahren in stark vereinfachter, schematischer Darstellung veranschaulicht und insgesamt mit 100 bezeichnet.

In dem Verfahren 100 werden ein oder mehrere Reaktoren eingesetzt, der oder die für eine Butandehydrierung 10 eingerichtet ist oder sind, und dem oder denen ein Butan enthaltender Reaktionseinsatz 1 zugeführt wird. Der oder die Reaktoren wird oder werden mit einem oder mehreren Brennern 11 beheizt.

Mittels des oder der Reaktoren, und damit unter Einsatz der darin durchgeführten Butandehydrierung 10, wird ein Butan, Butylen und Wasserstoff enthaltendes Komponentengemisch 2 gebildet und damit in dem Verfahren 100 bereitgestellt. Die Produktionsmenge beträgt beispielsweise ca. 50 kta. Das Komponentengemisch 2, oder nur ein Teil des Komponentengemischs 2, wird einer Abkühlung 20 und dabei insbesondere ein Wärmetausch mit dem Einsatzgemisch 1 unterworfen.

Nach der Abkühlung 20 bzw. Abkühlung wird das Komponentengemisch 2 oder dessen der Abkühlung unterworfener Teil, oder wiederum nur ein Teil hiervon, einer Verdichtung 30 unterworfen, wobei insbesondere ein mittels eines Elektromotors M angetriebener dreistufiger Turboverdichter in einem die drei Verdichterstufen einschließenden Gehäuse zum Einsatz kommen kann. Die Verdichtung erfolgt insbesondere auf ein Druckniveau von 12 bis 15 bar.

Nach der Verdichtung 30 wird das Komponentengemisch oder dessen der Verdichtung 30 unterworfener Teil, oder wiederum ein nur Teil hiervon, als erster Trenneinsatz einer ersten Membrantrennung 40 unterworfen. In dieser werden im dargestellten Beispiel ein gegenüber dem ersten Trenneinsatz an Wasserstoff angereichertes und an Butan und Butylen armes oder freies erstes Permeat 3 und ein gegenüber dem ersten Trenneinsatz an Wasserstoff abgereichertes, jedoch noch Wasserstoff und ansonsten Butan und Butylen enthaltendes erstes Retentat 4 gebildet. Im dargestellten Beispiel wird die erste Membrantrennung ohne Verwendung eines Butan enthaltenden Spülgases durchgeführt und damit das erste Permeat 3 nicht als ein mit Butan eines Spülgases beaufschlagtes Permeat gewonnen. Wie erläutert, werden aber auch Verfahrensvarianten in Erwägung gezogen, in denen genau dies erfolgt.

Die Membrantrennung 40 wird dabei im dargestellten Beispiel insbesondere derart vorgenommen, dass ein Druckniveau, auf dem das erste Permeat 3 gewonnen wird, gerade hoch genug ist, dass das erste Permeat 3, oder nur ein Teil hiervon, ohne weitere Verdichtung dem oder den Brennern 11 zugeführt und in diesem oder diesen als Heizgas eingesetzt werden kann. Reicht eine Menge des ersten Permeats 3 für eine Beheizung mittels des oder der Brenner 11 nicht aus, kann insbesondere vorgesehen sein, in der ersten Membrantrennung 30 eine Spülung mit einem brennbaren Spülgas, insbesondere Methan bzw. einem Methan enthaltenden Gasgemisch, insbesondere mit Erdgas, vorzunehmen. Das erste Permeat 3 kann auch in ein Heizgasnetz einer entsprechenden Anlage oder eines entsprechenden Anlagenverbunds eingespeist werden.

Das erste Retentat 4, oder nur ein Teil hiervon, wird als zweiter Trenneinsatz einer zweiten Membrantrennung 50 unterworfen, in der ein gegenüber dem zweiten Trenneinsatz unter Einsatz eines Butan enthaltenden Spülgases 5 ein zumindest den überwiegenden Anteil des in dem zweiten Trenneinsatz enthaltenen Wasserstoffs enthaltendes zweites Permeat 6 und ein zumindest den überwiegenden Teil des Butans und des Butylens aus dem zweiten Trenneinsatz enthaltendes zweites Retentat 7 gebildet werden. Das Spülgas 5 wird insbesondere deshalb verwendet, damit ungeachtet der in der ersten Membrantrennung 40 bereits erfolgten Reduzierung des Wasserstoffpartialdrucks eine ausreichende, d.h. vollständige oder im Wesentlichen vollständige, Wasserstoffabtrennung erfolgen kann.

Das zweite Permeat 6 umfasst aufgrund der erläuterten Bildung in der zweiten Membrantrennung 50 Butan des Spülgases 5 und Wasserstoff aus dem zweiten Trenneinsatz. Im dargestellten Beispiel wird also das zweite Permeat 6 als ein mit Butan des Spülgases 5 beaufschlagtes Permeat gewonnen. Das zweite Permeat 6, oder nur ein Teil hiervon, kann daher in besonders vorteilhafter Weise als Teil des Reaktionseinsatzes 1 in den oder die Reaktoren 10 zurückgeführt werden, da in der dort durchgeführten Butandehydrierung typischerweise Reaktionseinsätze zum Einsatz kommen, die einen bestimmten Anteil an Wasserstoff enthalten. Zu anderen Ausgestaltungen sei auf die obigen Erläuterungen verwiesen.

Daher kann im Rahmen der vorliegenden Erfindung die zweite Membrantrennung sowohl zur Abtrennung von Wasserstoff zur Gewinnung eines Butylenprodukts als auch zur Zudosierung von Wasserstoff zu einem Reaktionseinsatz 1 verwendet werden. Hierbei wird das zweite Permeat 6, oder dem als Teil des Reaktionseinsatzes 1 in den oder die Reaktoren 10 zurückgeführten Teil hiervon insbesondere ein überwiegend oder ausschließlich Butan enthaltender Frischeinsatz 8 zugegeben.

Das zweite Retentat 7 wird im dargestellten Beispiel einem oder mehreren weiteren Verfahrensschritten 60, insbesondere einer Trennung von Butan und Butylen und ggf. einer Abtrennung schwererer Komponenten zugeführt. In dem oder den weiteren Verfahrensschritten 60 kann, neben einer oder mehreren anderen, hier nicht gesondert veranschaulichten weiteren Fraktionen, insbesondere ein überwiegend oder ausschließlich Butylen enthaltendes Butylenprodukt 9 gebildet werden. Ferner kann in dem oder den weiteren Verfahrensschritten 60 insbesondere auch eine überwiegend oder ausschließlich Butan enthaltende Fraktion gewonnen werden, welche als das Spülgas 5 verwendet werden kann. Der oder die weiteren Verfahrensschritte 60 kann bzw. können auch insbesondere Teil eines Dampfspaltprozesses mit entsprechend zugeordneten Trennschritten sein.

## Patentansprüche

1. Verfahren (100) zur Herstellung eines Butylenprodukts (9), bei dem unter Einsatz einer Butandehydrierung (10), der ein Butan und Wasserstoff enthaltender Reaktionseinsatz (1) unterworfen wird, ein Butan, Butylen und Wasserstoff enthaltendes Komponentengemisch (2) bereitgestellt wird,
- wobei das Komponentengemisch (2) oder ein Teil hiervon als ein erster Trenneinsatz einer ersten Membrantrennung (40) unterworfen wird, mittels derer ein gegenüber dem ersten Trenneinsatz an Wasserstoff angereichertes erstes Permeat (3) sowie ein gegenüber dem ersten Trenneinsatz an Wasserstoff abgereichertes, Wasserstoff, Butan und Butylen enthaltendes erstes Retentat (4) gebildet werden,
- wobei das erste Retentat (4) oder ein Teil hiervon als zweiter Trenneinsatz einer zweiten Membrantrennung (50) unterworfen wird, in der ein zumindest den überwiegenden Teil des Wasserstoffs des zweiten Trenneinsatzes enthaltendes zweites Permeat (6) sowie ein zumindest den überwiegenden Teil des Butans und des Butylens des zweiten Trenneinsatzes enthaltendes zweites Retentat gebildet werden,
- wobei die erste Membrantrennung (40) unter Einsatz eines Butan enthaltenden Spülgases (5) durchgeführt wird und das erste Permeat (3) als mit Butan des Spülgases (5) beaufschlagtes Permeat (3) erhalten wird und/oder die zweite Membrantrennung (50) unter Einsatz des Butan enthaltenden Spülgases (5) durchgeführt wird und das zweite Permeat (6) als mit Butan des Spülgases (5) beaufschlagtes Permeat (6) erhalten wird,
- wobei das mit Butan des Spülgases (5) beaufschlagte erste Permeat (3) und/oder das mit Butan des Spülgases beaufschlagte zweite Permeat (3) oder ein oder mehrere Teile hiervon bei der Bildung des Reaktionseinsatzes (1) verwendet wird.

2. Verfahren nach Anspruch 1, bei dem der Reaktionseinsatz (1) 45 bis 95 Volumenprozent Butan und 1 bis 50 Volumenprozent Wasserstoff aufweist.

3. Verfahren nach Anspruch 1 oder 2, bei dem das Komponentengemisch (2) und der erste Trenneinsatz 40 bis 60 Volumenprozent Butan, 20 bis 30 Volumenprozent Butylen und 25 bis 30 Volumenprozent Wasserstoff aufweisen.

4. Verfahren nach einem der vorstehenden Ansprüche, bei dem das erste Retentat und der zweite Trenneinsatz 10 bis 20 Volumenprozent Wasserstoff aufweisen.

5. Verfahren nach einem der vorstehenden Ansprüche, bei dem das zweite Retentat 5 bis 15 Volumenprozent Wasserstoff aufweist.

6. Verfahren nach einem der vorstehenden Ansprüche, bei dem das Komponentengemisch (2) oder dessen der ersten Membrantrennung (40) unterworfener Teil einer Abkühlung (20) und Verdichtung (30) unterworfen wird, bevor es oder er der Membrantrennung (40) unterworfen wird.

7. Verfahren nach Anspruch 6, bei dem die Verdichtung (30) auf ein Druckniveau von 2 bis 38 bar vorgenommen wird.

8. Verfahren nach Anspruch 6 oder 7, bei dem die Verdichtung (30) unter Verwendung eines oder mehrerer mehrstufiger Turboverdichter, insbesondere mit zwei oder weniger Zwischenkühlstufen, vorgenommen wird.

9. Verfahren nach einem der vorstehenden Ansprüche, bei dem das zweite Retentat oder ein Teil hiervon, insbesondere auf einem Druckniveau von 1 bis 38 bar, einem oder mehreren weiteren Verfahrensschritten (60) zugeführt wird.

10. Verfahren nach Anspruch 9, bei dem der oder die weiteren Verfahrensschritte (60) einen Dampfspaltprozess oder einen oder mehrere dem Dampfspaltprozess zugeordnete Trennschritte umfassen.

11. Verfahren nach einem der vorstehenden Ansprüche, bei dem nur die erste Membrantrennung (40) unter Einsatz des Butan enthaltenden Spülgases (5) durchgeführt wird und nur das erste Permeat (3) als mit Butan des Spülgases (5) beaufschlagtes Permeat (3) erhalten wird oder bei dem nur die zweite Membrantrennung (50) unter Einsatz des Butan enthaltenden Spülgases (5) durchgeführt wird und nur das zweite Permeat (6) als mit Butan des Spülgases (5) beaufschlagtes Permeat (6) erhalten wird, wobei das jeweils nicht mit Butan des Spülgases (5) beaufschlagte Permeat (3, 6) oder ein Teil hiervon in einem oder mehreren für die Butandehydrierung (10) verwendeten Reaktoren zur Beheizung verfeuert wird.

12. Verfahren nach Anspruch 11, bei dem das jeweils nicht mit Butan des Spülgases (5) beaufschlagte Permeat (3, 6) in der Membrantrennung (40, 50) auf einem Druckniveau von 3 bis 7 bar bereitgestellt wird.

13. Anlage zur Herstellung eines Butylenprodukts (9), mit einem oder mehreren Reaktoren, der oder die dafür eingerichtet sind, unter Einsatz einer Butandehydrierung (10), dem ein Butan und Wasserstoff enthaltender Reaktionseinsatz (1) unterworfen wird, ein Butan, Butylen und Wasserstoff enthaltendes Komponentengemisch (2) bereitzustellen,
- Mitteln, die dafür eingerichtet sind, das Komponentengemisch (2) oder einen Teil hiervon als einen ersten Trenneinsatz einer ersten Membrantrennung (40) zu unterwerfen, mittels derer ein gegenüber dem ersten Trenneinsatz an Wasserstoff angereichertes erstes Permeat (3) sowie ein gegenüber dem ersten Trenneinsatz an Wasserstoff abgereichertes, Wasserstoff, Butan und Butylen enthaltendes erstes Retentat (4) gebildet werden,
- Mitteln, die dafür eingerichtet sind, das erste Retentat (4) oder einen Teil hiervon als einen zweiten Trenneinsatz einer zweiten Membrantrennung (50) zu unterwerfen, in der ein zumindest den überwiegenden Teil des Wasserstoffs des zweiten Trenneinsatzes und des Butans des Spülgases enthaltendes zweites Permeat (6) sowie ein zumindest den überwiegenden Teil des Butans und des Butylens des zweiten Trenneinsatzes enthaltendes zweites Retentat gebildet werden,
- Mitteln, die dafür eingerichtet sind, die erste Membrantrennung (40) unter Einsatz eines Butan enthaltenden Spülgases (5) durchzuführen und das erste Permeat (3) als mit Butan des Spülgases (5) beaufschlagtes Permeat (3) zu erhalten und/oder die zweite Membrantrennung (50) unter Einsatz des Butan enthaltenden Spülgases (5) durchzuführen und das zweite Permeat (6) als mit Butan des Spülgases (5) beaufschlagtes Permeat (6) zu erhalten,
- Mitteln, die dafür eingerichtet sind, das mit Butan des Spülgases (5) beaufschlagte erste Permeat (3) und/oder das mit Butan des Spülgases beaufschlagte zweite Permeat (3) oder ein oder mehrere Teile hiervon bei der Bildung des Reaktionseinsatzes (1) zu verwendet.

14. Anlage nach Anspruch 13, die zur Durchführung eines Verfahrens nach einem der vorstehenden Ansprüche eingerichtet ist.
